# EUROPEAN PATENT APPLICATION

(11) **EP 3 858 390 A1**
(43) Date of publication of application: **04.08.2021**
(21) Application number: 21153348.4
(22) Date of filing: 25.01.2021
(51) Int. Cl.: A61L 2/10, C02F 1/32

(54) **FLUID STERILIZATION DEVICE AND FLUID STERILIZATION UNIT**

(30) Priority: 28.01.2020 JP 2020011551
(71) Applicant: STANLEY ELECTRIC CO., LTD., Tokyo 153-8636 (JP)
(72) Inventor: TANAKA, Hideaki, Tokyo, 153-8636 (JP); KATO, Hiroyuki, Tokyo, 153-8636 (JP); SHINNO, Kazuhisa, Tokyo, 153-8636 (JP)
(74) Representative: Emde, Eric

(57) **Abstract**

There are provided a fluid sterilization device (1, 2, 3) and a fluid sterilization unit (500, 600, 700) including the fluid sterilization device(s) (1, 2, 3), which can reduce the generation of bubbles and a stagnant layer as much as possible even if the fluid is allowed to flow through the tube. The fluid sterilization device (1, 2, 3) according to the present invention includes: a main tube portion (10, 60, 70) including an inlet port (11) for a fluid provided on one side of both ends opposite to each other in an axis direction of the main tube portion (10, 60, 70) and an outlet port (12) for the fluid provided on the other side; a communication tube portion (20) connected to a side wall (10S, 10S1) of the main tube portion (10, 60, 70) so as to communicate with an inside of the main tube portion (10, 60, 70); a light source portion (30) accommodated in the communication tube portion (20) so as to face the inside of the main tube portion (10, 60, 70), the light source portion (30) being configured to irradiate the fluid flowing through the main tube portion (10, 60, 70) with ultraviolet light; and a cap portion (40) having an ultraviolet ray transmitting ability, the cap portion (40) being configured to separate the light source portion (30) and the main tube portion (10, 60, 70). In the fluid sterilization device (1, 2, 3) with this configuration, the cap portion (40) has a distal end wall (41) that faces the light source portion (30), and the distal end wall (41) may be disposed along the side wall (10S, 10S1) connected to the communication tube portion (20), or may be disposed inside the main tube portion (10, 60, 70) from the side wall (10S, 10S1).

## Description

### Technical Field

The present invention relates to a fluid sterilization device and a fluid sterilization unit.

### Background Art

In the past, various products have been proposed, which irradiate ultraviolet light is irradiated to sterilize a fluid. Examples of such a fluid may include beverages, raw water for food, and cooling or washing water for industrial use. As an invention relating to these liquid sterilization products, a flow water sterilization module to be provided in a flow path of a fluid is disclosed in JP2019-018198A.

The flow water sterilization module disclosed in JP2019-018198A includes a flow channel tube that has an internal space through which water as a raw material fluid flows, and a light source for irradiating the internal space with ultraviolet light. The flow water sterilization module further includes a columnar heat dissipation member that extends from one end portion of the flow channel tube toward the internal space. In this structure, the light source is attached to the distal end of the columnar heat dissipation member.

However, in the flow water sterilization module disclosed in JP2019-018198A, the entire light source and most (or all) of the columnar heat dissipation member to which the light source is attached protruded into the internal space of the flow channel tube. The light source and the columnar heat dissipation member are disposed at the same end (the one end) as the inlet/outlet portion (the first inlet/outlet portion disclosed in JP2019-018198A) and face the inlet/outlet portion.

If bubbles are contained in the fluid flowing into the internal space of the flow channel tube from the inlet/outlet portion, the fluid collides with the light source and the columnar heat dissipation member, so that bubbles are attached to the vicinity of the light source and the columnar heat dissipation member. Thus, since the bubbles are interposed between the light source and the fluid, ultraviolet light to be irradiated to the fluid from the light source is blocked. As a result, the sterilization efficiency for the fluid is reduced.

Further, according to the technique disclosed in JP2019-018198A, due to the collision between the fluid and the light source and the columnar heat dissipation member, a vortex flow occurs in the fluid after the collision (for example, see paragraph 0020 of JP2019-018198A, etc.). As a result, since a stagnant layer is generated in the vicinity of the heat dissipation member, pressure loss occurs in the fluid flowing into the internal space of the flow channel tube from the inlet/outlet portion. Therefore, the smooth flow of the fluid in the flow channel tube is inhibited.

### Summary

In view of the above-mentioned problems, an object of the present invention is to provide a fluid sterilization device which is capable of reducing the generation of bubbles and a stagnant layer as much as possible even when a fluid is allowed to flow through a tube, and a fluid sterilization unit including the fluid sterilization device.

In order to solve the above-mentioned problems, a fluid sterilization device according to the present invention includes: a main tube portion including an inlet port for a fluid provided on one side of both ends opposite to each other in an axis direction of the main tube portion and an outlet port for the fluid provided on the other side; a communication tube portion connected to a side wall of the main tube portion so as to communicate with an inside of the main tube portion; a light source portion accommodated in the communication tube portion so as to face the inside of the main tube portion, the light source portion being configured to irradiate the fluid flowing through the main tube portion with ultraviolet light; and a cap portion having an ultraviolet ray transmitting ability, the cap portion being configured to separate the light source portion and the main tube portion. In the fluid sterilization device with this configuration, the cap portion has a distal end wall that faces the light source portion, and the distal end wall may be disposed along the side wall connected to the communication tube portion, or may be disposed inside the main tube portion from the side wall.

In the fluid sterilization device according to this aspect of the present invention, the inlet port for the fluid is on one side of ends of the main tube portion opposite to each other in the axis direction thereof, and the outlet port for the fluid is on the other side. Therefore, this configuration can allow the fluid to flow through the main tube portion without greatly changing the traveling direction of the fluid across both ends of the main tube portion. As a result, generation of bubbles and stagnant layer in the main tube portion can be greatly reduced.

In addition, in the fluid sterilization device according to this aspect of the present invention, the distal end wall of the cap portion that separates the light source portion and the main tube portion in the communication tube portion (wall portion that faces the light source portion) may be disposed along the side wall of the main tube portion that is connected to the communication tube portion or may be disposed inside the main tube portion from the side wall. Therefore, concave spaces, into which the fluid and bubbles flowing through the main tube portion may flow, are not formed in the boundary portion between the light source portion and the main tube portion (the foregoing distal end wall). Thus, stagnant layer and bubbles are less likely to be generated in the boundary portion between the light source portion and the main tube portion.

Therefore, the fluid sterilization device according to this aspect of the present invention can reduce the pressure loss which would act as a resistance against the fluid flowing through the main tube portion. In addition, the fluid sterilization device according to this aspect of the present invention can prevent the ultraviolet light irradiated from the light source portion to the fluid from being blocked by bubbles, thereby effectively preventing the sterilization efficiency from being reduced.

In the fluid sterilization device according to the present invention, the main tube portion may preferably include a first tube portion that includes a communication region with the communication tube portion, and a second tube portion connected to an end of the first tube portion on a side closer to the inlet port. In this configuration, the second tube portion may preferably have a diameter that increases from the side closer to the inlet port toward the first tube portion.

In the fluid sterilization device according to this aspect of the present invention, the second tube portion expands in diameter as it approaches the first tube portion from the inlet port side. Increasing the diameter of the second tube portion in this manner can reduce the flow rate of the fluid passing through the second tube portion as the fluid approaches the first tube portion. At this time, the first tube portion is connected to the communication tube portion that accommodates the light source portion. Therefore, the fluid sterilization device according to this aspect of the present invention can reduce the flow rate of the fluid that flows in the vicinity of the light source portion, and also can increase the irradiation amount of ultraviolet light. As a result, the fluid sterilization device can increase the sterilization efficiency.

Further, in the fluid sterilization device according to the present invention, in a vertical cross-sectional view of the second tube portion, the second tube portion may preferably include a side wall region on one side disposed closer to the light source portion, the side wall region on the one side extending along the axis direction of the main tube portion, and a side wall region on the other side, which faces the side wall region on the one side, the side wall region on the other side being inclined so as to increase the distance to the side wall region on the one side as approaching the first tube portion from the inlet port side.

The fluid sterilization device according to this aspect of the present invention can control the flow rate of the fluid passing through the second tube portion to the first tube portion so that the flow rate thereof is made higher as the fluid is closer to the side wall region on the one side (the side wall region disposed close to the light source portion) and made lower as the fluid is closer to the side wall region on the other side (the side wall region facing the side wall region on the one side). At this time, the intensity of the ultraviolet light irradiated from the light source portion becomes stronger toward the side wall region on the one side, and becomes weaker toward the side wall region on the other side. Therefore, the fluid sterilization device according to this aspect of the present invention can irradiate the fluid flowing through the main tube portion with ultraviolet light of high intensity in the region where the flow rate is high, while it can irradiate the fluid with ultraviolet light of low intensity in the region where the flow rate is low. Thus, the fluid sterilization device can irradiate the fluid with a sufficient amount of ultraviolet light regardless of the distance from the light source portion. As a result, the sterilization efficiency for the fluid can be increased.

Further, the fluid sterilization unit according to the present invention includes: a plurality of fluid sterilization devices with the foregoing configuration. In two adjacent fluid sterilization devices of these, an inlet port of one of the two adjacent fluid sterilization devices and an outlet port of the other of the two adjacent fluid sterilization devices are connected to each other.

In the fluid sterilization unit according to this aspect of the present invention, the plurality of fluid sterilization devices can be linked so that the inlet port of one of two adjacent fluid sterilization devices and the outlet port of the other one of the two adjacent fluid sterilization devices are connected to each other. Therefore, the fluid can be irradiated with a sufficient amount of ultraviolet light. As a result, the sterilization efficiency for the fluid can be increased.

Thus, the present invention can provide the foregoing fluid sterilization device and the above-described fluid sterilization unit including the fluid sterilization devices, which can reduce the generation of bubbles and a stagnant layer as much as possible even if the fluid is allowed to flow through the tube.

### Brief Description of Drawings

These and other characteristics, features, and advantages of the present invention will become clear from the following description with reference to the accompanying drawings, wherein:
FIG. 1A, FIG. 1B, and FIG. 1C are a plan view, a side view, and a vertical cross-sectional view, respectively, of a fluid sterilization device as a first embodiment according to the present invention.
FIG. 2 is a vertical cross-sectional view illustrating a communication tube portion communicating with a main tube portion of the fluid sterilization device of the first embodiment, and a light source portion accommodated in the communication tube portion.
FIG. 3 is a vertical cross-sectional view of a fluid sterilization device as a second embodiment according to the present invention.
FIG. 4 is a vertical cross-sectional view of a fluid sterilization device as a third embodiment according to the present invention.
FIG. 5 is a vertical cross-sectional view of a fluid sterilization unit according to the present invention.
FIG. 6 is a vertical cross-sectional view of another fluid sterilization unit according to the present invention.
FIG. 7 is a vertical cross-sectional view of yet another fluid sterilization unit according to the present invention.

### Description of Exemplary Embodiments

### First Embodiment:

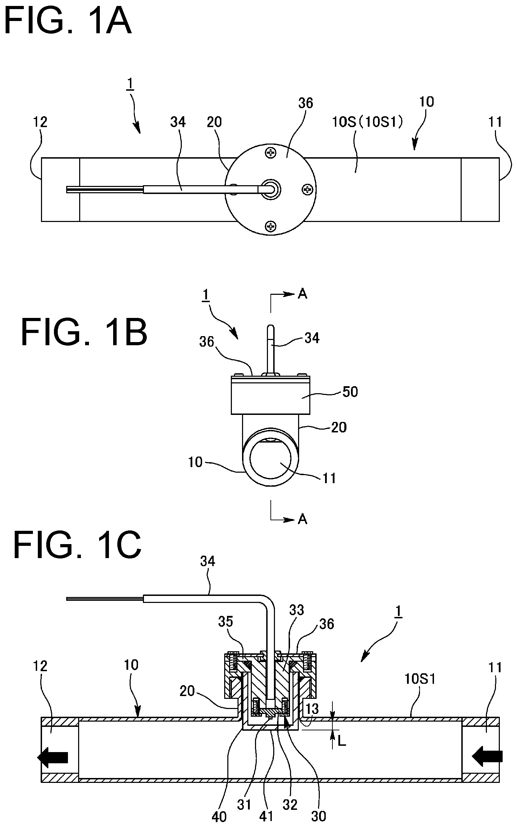

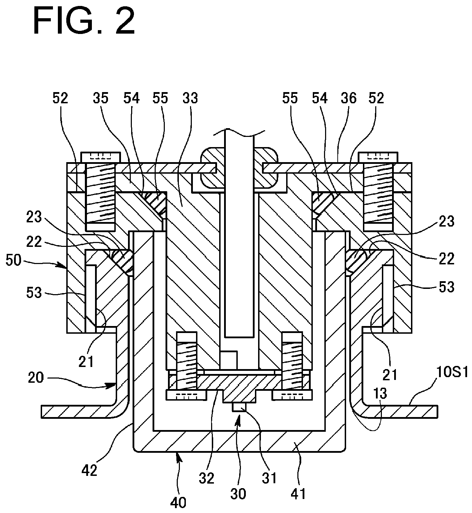

A description will now be made below to fluid sterilization devices of the present invention in detail with reference to the accompanying drawings in accordance with exemplary embodiments. First, referring to FIGS. 1A to 1C and 2, a fluid sterilization device 1 as a first embodiment according to the present invention will be described. FIG. 1A is a plan view of the fluid sterilization device 1, FIG. 1B is a side view thereof, and FIG. 1C is a vertical cross-sectional view thereof - a cross-sectional view taken along line A-A of FIG. 1B. FIG. 2 is a vertical cross-sectional view illustrating a communication tube portion 20 that communicates with a main tube portion 10 of the fluid sterilization device 1, and a light source portion 30 that is accommodated in the communication tube portion 20.

For example, as shown in FIG. 1C, the fluid sterilization device 1 according to the present embodiment includes the main tube portion 10 through which a fluid flows, the communication tube portion 20 that communicates with the inside of the main tube portion 10 by connecting it with a side wall 10S of the main tube portion 10 (a side wall 10S1 in FIG. 1C), the light source portion 30 that is accommodated in the communication tube portion 20, and a cap portion 40 that separates the light source portion 30 and the main tube portion 10.

The main tube portion 10 in the present embodiment is a long cylindrical body, but is not limited thereto. The form of the main tube portion 10 may be, for example, a long prismatic cylinder or the like. Further, the main tube portion 10 in the present embodiment is made of stainless steel, but is not limited thereto. Examples of the material of the main tube portion 10 may include a metal other than stainless steel and a resin such as tetrafluoroethylene (PTFE). As described later, the main tube portion 10 in the present embodiment is in thermal contact with the light source portion 30 via the communication tube portion 20 and a frame body 50, which will be described later, both made of metal. Therefore, the metal-made main tube portion 10 can efficiently dissipate heat transferred from the light source portion 30 to the outside of the main tube portion 10, thereby effectively cool the light source portion 30.

An inlet port 11 is provided at one of both opposite ends in the axis direction of the main tube portion 10 (long axis direction). An outlet port 12 is provided at the other end. A fluid to be sterilized flows into the main tube portion 10 from the inlet port 11, flows through the main tube portion 10, and then flows out of the main tube portion 10 through the outlet port 12. The light source portion 30 is accommodated in the communication tube portion 20 from the upper side thereof in FIG. 1C so as to face the inside of the main tube portion 10 (i.e., the fluid flowing through the main tube portion 10), and thus, the fluid is irradiated with ultraviolet light from the light source portion 30. This causes the fluid to be sterilized. Examples of the fluid to flow through the main tube portion 10 are not limited thereto, but may include drinking water such as mineral water and carbonated water, food raw material water, and cooling or washing water for industrial use.

As shown in FIGS. 1A to 1C, the fluid flowing through the main tube portion 10 flows substantially linearly from the inlet port 11 to the outlet port 12 opposite to each other in the axis direction. That is, the fluid flowing through the main tube portion 10 flows without greatly changing the traveling direction. Therefore, the fluid sterilization device 1 can suppress the generation of a stagnant layer, which would otherwise be generated due to bubbles and vortex flow in the main tube portion 10. As a result, the fluid in the main tube portion 10 can smoothly flow therethrough.

The communication tube portion 20 in the present embodiment is a cylindrical body that extends radially outward from the main tube portion 10 from the peripheral edge of a through hole 13 provided in the side wall 10S1 of the main tube portion 10. Further, the inside of the main tube portion 10 and the inside of the communication tube portion 20 communicate with each other through the through hole 13.

The communication tube portion 20 may be made of metal such as stainless steel or aluminum, or may be made of a resin such as PTFE. As described later, it is preferable for the communication tube portion 20 to be made of metal capable of efficiently dissipating heat transferred from the light source portion 30 to the main tube portion 10 since the communication tube portion 20 serves as a member being in thermal contact with the light source portion 30.

The light source portion 30 is accommodated in the communication tube portion 20. The light source portion 30 is insulated with the cap portion 40. Thus, the cap portion 40 separates the main tube portion 10 and the light source portion 30, and as a result of this, the fluid flowing through the main tube portion 10 enters the communication tube portion 20, thereby avoiding a situation of being in contact with the light source portion 30. Hereinafter, the communication tube portion 20, the light source portion 30, and the cap portion 40 will be described in details with reference to FIG. 2.

As shown in FIG. 2, the light source portion 30 includes a light emitting element 31 capable of irradiating ultraviolet light, a substrate 32 for mounting the light emitting element 31, and a heat sink 33 for mounting the substrate 32 and dissipating heat transferred from the light emitting element 31 through the substrate 32. The light emitting element 31 is mounted on the surface of the substrate 32 opposed to the main tube portion 10 to face the fluid flowing through the main tube portion 10. The light emitting element 31 is electrically connected to a power supply cable 34 via the substrate 32.

The type of the light emitting element 31 is not particularly limited, and for example, a semiconductor light emitting element such as a light emitting diode (LED) or a laser diode may be used. In the light source portion 30 shown in FIG. 2, the number of light emitting elements 31 mounted on the substrate 32 is one, but may be two or more.

The light source portion 30 in the present embodiment is supported by the frame body 50 having a substantially cylindrical shape. The frame body 50 for supporting the light source portion 30 is detachably mounted to the communication tube portion 20. More specifically, when the light source portion 30 is inserted into the hollow area formed in the center of the frame body 50, the flange surface 35 of the heat sink 33 is superimposed on the upper surface 52 of the frame body 50. Thereafter, the flange surface 35 is screwed to the upper surface 52 of the frame body 50. As shown in FIG. 2, the screwed flange surface 35 may be covered with a metal lid 36.

Further, a male screw groove 53 is formed at the lower end of the frame body 50, and a female screw groove 21 screwed into the male screw groove 53 is formed at the upper end of the outer surface of the communication tube portion 20. After the lower end of the frame body 50 and the upper end of the communication tube portion 20 are in contact with each other, the frame body 50 is rotated, so that the male screw groove 53 and the female screw groove 21 are engaged with each other. As a result, the frame body 50 is screwed to the communication tube portion 20, and thus, the light source portion 30 is accommodated in the communication tube portion 20.

Thus, since the light source portion 30 is detachably provided in the communication tube portion 20 via the frame body 50, it is possible to easily perform the operation of replacing the existing light source portion 30 to another new light source portion 30. The replacement operation of the light source portion 30 in the present embodiment is to mount the frame body 50, to which the light source portion 30 has been attached, with respect to the communication tube portion 20 that extends outward from the straight tubular main tube portion 10. Therefore, when the frame body 50 is mounted to the communication tube portion 20, there is no site that interferes with the operator's hand, and thus it is possible to perform the replacement operation of the light source portion 30 without stress and also quickly perform the replacement operation.

Further, as shown in FIG. 2, a triangular groove 54 is formed at the upper end of the frame body 50. An O-ring 55 for sealing a joint portion between the light source portion 30 and the frame body 50 is fitted to the triangular groove 54. Similarly, a triangular groove 22 is formed at the upper end of the inner surface of the communication tube portion 20. In the triangular groove 22, an O-ring 23 for sealing a joint portion between the communication tube portion 20 and the cap portion 40 and a joint portion between the communication tube portion 20 and the frame body 50 is fitted.

The material for the frame body 50 is not particularly limited, and in order to efficiently dissipate heat transferred from the heat sink 33 of the light source portion 30 to the communication tube portion 20, the frame body may be preferably made of metal. Thus, similar to the frame body 50, the main tube portion 10 and the communication tube portion 20 are made of metal, so that it is possible to efficiently transmit heat from the heat sink 33 to the fluid flowing through the main tube portion 10. As a result, it is possible to effectively cool the light source portion 30.

The cap portion 40 is made of a material that has ultraviolet transmitting ability such as quartz, perfluoroalkoxyalkane (PFA), or a perfluoroethylene propene copolymer (FEP), and is a member which is fitted into a hollow region inside the communication tube portion 20 so as to coat the light source portion 30. The cap portion 40 in the present embodiment is a cylindrical body having an opening at one end, and includes a distal end wall 41 facing the light emitting element 31 of the light source portion 30, and a side peripheral wall 42 extending substantially perpendicularly continuous with the peripheral edge of the distal end wall 41. The distal end wall 41 in the present embodiment is a substantially parallel plane wall to the side wall 10S1 of the main tube portion 10. However, the shape of the distal end wall 41 is not limited to this, and another form of the distal end wall 41 may be a curved wall curving inward (protruding) toward the inside of the main tube portion 10 such as a hemisphere or semi-elliptical sphere.

A distal end wall 41 of the cap portion 40 is disposed inside the main tube portion 10 from the side wall 10S1. For example, in the case of the mode shown in FIG. 1C, the distal end wall 41 of the cap portion 40 is disposed inside the main tube portion 10 by a distance L from the side wall 10S1. The distal end wall 41 of the cap portion 40 and the side wall 10S1 with such a positional relationship can prevent the formation of a concave space, in which the fluid and bubbles flowing through the main tube portion 10 would flow, in the boundary portion between the light source portion 30 and the main tube portion 10 (distal end wall 41).

This configuration can also prevent the generation of a stagnant layer and bubbles in the boundary portion between the light source portion 30 and the main tube portion 10. As a result, the fluid can smoothly flow through the main tube portion 10, and the ultraviolet light, which is irradiated from the light source portion 30 to the fluid, is prevented from being blocked by air bubbles. Thus, it is possible to effectively prevent a situation in which the sterilization efficiency for the fluid is reduced due to such bubbles.

Herein, the positional relationship between the distal end wall 41 and the side wall 10S1 is not limited to the foregoing structure. For example, the distal end wall 41 may be disposed along the side wall 10S1, i.e., the distance L between the distal end wall 41 and the side wall 10S1 described above is zero (0). Thus, the distal end wall 41 and the side wall 10S1 may be disposed at substantially the same height position. Even with the distal end wall 41 and the side wall 10S1 being at such a positional relationship, a concave space is not formed in the boundary portion between the light source portion 30 and the main tube portion 10 (at the distal end wall 41). As a result, it is possible to suppress a situation in which a stagnant layer is generated or bubbles remain in the vicinity of the boundary portion, i.e., the distal end wall 41.

### Second Embodiment:

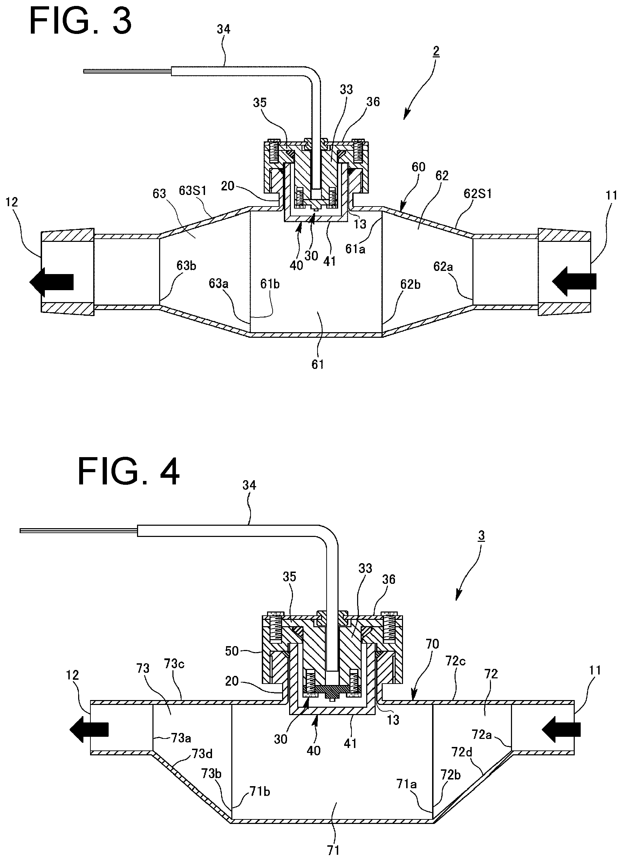

Next, a second embodiment of the fluid sterilization device of the present invention will be described with reference to FIG. 3. FIG. 3 is a vertical cross-sectional view of the fluid sterilization device 2 according to the second embodiment. A description of portions/regions that do not change from those of the first embodiment is omitted.

A portion different in the second embodiment from the first embodiment described above is the form of the main tube portion 60. The main tube portion 10 in the first embodiment is a straight tubular cylindrical body having no inclined portion at substantially the same diameter over the outlet port 12 from the inlet port 11. In contrast, the main tube portion 60 in the second embodiment, as shown in FIG. 3, includes a straight tubular first tube portion 61 located in the center in the axis direction of the main tube portion 60, a frustoconical second tube portion 62 which is disposed on the inlet port 11 side from the first tube portion 61, the second tube portion 62 expanding in diameter as approaching the first tube portion 61, and a third tube portion 63 which is disposed on the outlet port 12 side from the first tube portion 61, the third tube portion 63 decreasing in diameter as away from the first tube portion 61.

The first tube portion 61 is connected to the communication tube portion 20, so that both interior spaces are communicated with each other. The second tube portion 62 is continuous with one end portion 61a of the first tube portion 61 (the end portion closer to the inlet port 11). The third tube portion 63 is continuous with the other end portion 61b of the first tube portion 61 (the end portion closer to the outlet port 12).

According to the second embodiment, the diameter of the second tube portion 62 increases as it approaches the first tube portion 61. Therefore, the flow rate of the fluid flowing into the main tube portion 60 from the inlet port 11 can be reduced as it approaches the first tube portion 61. Thus, this configuration can slow the flow rate of the fluid in the vicinity of the light source portion 30 facing the inside of the first tube portion 61, so that the fluid can be irradiated with ultraviolet light in an increased amount of light. As a result, the sterilization efficiency for the fluid can be increased.

As shown in FIG. 3, the second tube portion 62 is gradually enlarged in diameter in a slope shape from one end portion 62a closer to the inlet port 11 toward the other end portion 62b closer to the first tube portion 61. This configuration can allow the fluid to flow through the second tube portion 62 without greatly changing the traveling direction. As a result, the generation of the stagnant layer in the second tube portion 62 is suppressed, and the fluid can thus smoothly flow.

In contrast, the third tube portion 63 is a frustoconical cylindrical body having a reduced diameter as approaching the outlet port 12 from the first tube portion 61. As shown in FIG. 3, the third tube portion 63 is gradually reduced in diameter in a slope shape from one end portion 63a closer to the first tube portion 61 to the other end portion 63b closer to the outlet port 12. This configuration can allow the fluid to flow through the third tube portion 63 without greatly changing the traveling direction. As a result, the generation of the stagnant layer in the third tube portion 63 is suppressed, and the fluid can thus smoothly flow.

### Third Embodiment:

Next, a third embodiment of the fluid sterilization device of the present invention will be described with reference to FIG. 4. FIG. 4 is a vertical cross-sectional view of a fluid sterilization device 3 of the third embodiment. A description of portions/regions that do not change from those of the foregoing embodiments is omitted.

In the third embodiment, the shapes of a second tube portion 72 and a third tube portion 73 of a main tube portion 70 are different from those of the corresponding portions in the second embodiment. As described above, the second tube portion 62 and the third tube portion 63 in the second embodiment are truncated conical cylindrical bodies. On the other hand, the second tube portion 72 and the third tube portion 73 of the third embodiment are truncated half-conical cylindrical bodies corresponding to half portions obtained by longitudinally cutting the truncated cone from its top surface to its bottom surface.

More specifically, one end portion 72a of the second tube portion 72 corresponding to the upper surface of the truncated half-conical cylindrical body is disposed closer to the inlet port 11. The other end portion 72b of the second tube portion 72 corresponding to the bottom surface of the truncated half-conical cylindrical body is continuous with one end portion 71a of the first tube portion 71, i.e., the end portion thereof closer to the inlet port 11.

Thus, in the vertical cross-sectional view of the second tube portion 72 (see FIG. 4), the second tube portion 72 includes a side wall region 72c on one side (i.e., one side wall region 72c) disposed closer to the light source portion 30 (on the upper side in FIG. 4), where the one side wall portion 72c is not inclined, but extends along the axis direction of the main tube portion 70. In contrast, in a vertical cross-sectional view of the second tube portion 72 (see FIG. 4), the second tube portion 72 includes a side wall region 72d on the other side (i.e., the other side wall region 72d) facing the one side wall region 72c. The other side wall region 72d is inclined so as to increase the distance to the one side wall region 72c as approaching the first tube portion 71.

The second tube portion 72 with such a structure can increase the flow rate of the fluid passing through the second tube portion 72 and reaching the first tube portion 71 as the fluid approaches the one side wall region 72c, and can decrease the flow rate thereof as the fluid approaches the other side wall region 72d. In this configuration, the intensity of the ultraviolet light irradiated from the light source portion 30 to the area closer to the one side wall region 72c becomes stronger (higher) whereas the intensity thereof to the area closer to the other side wall region 72c becomes weaker (lower). Therefore, according to the third embodiment, the fluid flowing through the main tube portion 70 can be irradiated with ultraviolet light of high intensity in the region where the flow rate is high, while the fluid can be irradiated with ultraviolet light of low intensity in the region where the flow rate is low. Thus, the fluid sterilization device can irradiate the fluid with a sufficient amount of ultraviolet light regardless of the distance from the light source portion. As a result, the sterilization efficiency for the fluid can be increased.

The other side wall region 72d in the second tube portion 72 is gradually inclined in a slope shape from one end portion 72a closer to the inlet port 11 toward the other end portion 72b closer to the first tube portion 71. This configuration can allow the fluid to flow through the second tube portion 72 without greatly changing the traveling direction. As a result, the generation of the stagnant layer in the second tube portion 72 is suppressed, and the fluid can smoothly flow.

Furthermore, in the third tube portion 73, one end portion 73a of the third tube portion 73 corresponding to the upper surface of the truncated half-conical cylindrical body is disposed closer to the outlet port 12. The other end portion 73b of the third tube portion 73 corresponding to the bottom surface of the truncated half-conical cylindrical body is continuous with the other end portion 71b of the first tube portion 71, i.e., the end portion closer to the outlet port 12.

Thus, in the vertical cross-sectional view of the third tube portion 73 (see FIG. 4), the third tube portion 73 include one side wall region 73c on one side (i.e., one side wall region 73c) disposed closer to the light source portion 30 (on the upper side in FIG. 4), where the one side wall region 72c is not inclined, but extends along the axis direction of the main tube portion 70. In contrast, in the vertical cross-sectional view of the third tube portion 73 (see FIG. 4), the third tube portion 73 includes a side wall region 73d on the other side (i.e., the other side wall region 73d) facing the one side wall region 73c. The other side wall region 73d is inclined so as to decrease the distance to the one side wall region 73c, as it is distanced from the first tube portion 71.

Thus, the other side wall region 73d in the third tube portion 73 is gradually inclined in a slope shape from the other end portion 73b closer to the first tube portion 71 toward the one end portion 73a closer to the outlet port 12. This configuration can allow the fluid to flow through the third tube portion 73 without greatly changing the traveling direction. As a result, the generation of the stagnant layer in the third tube portion 73 is suppressed, and the fluid can thus smoothly flow.

### Fluid Sterilization Unit:

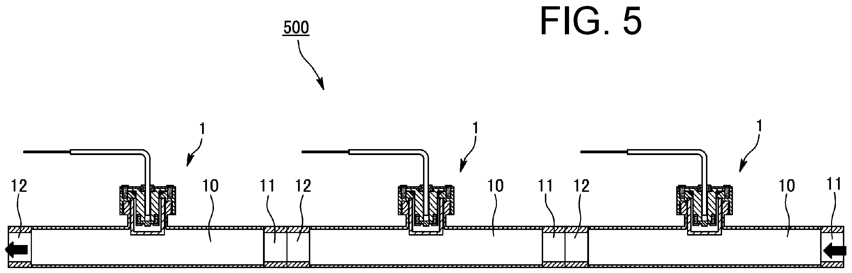

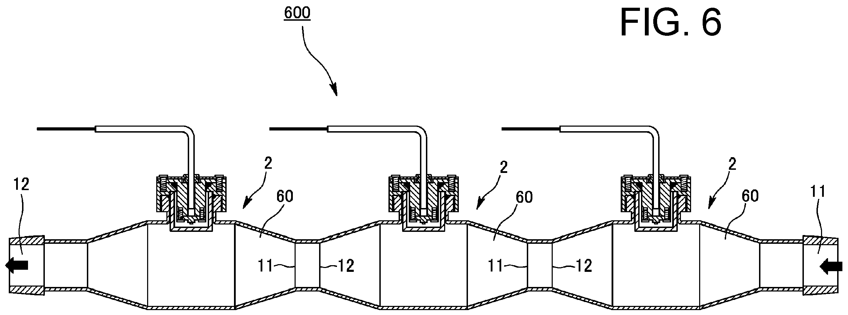

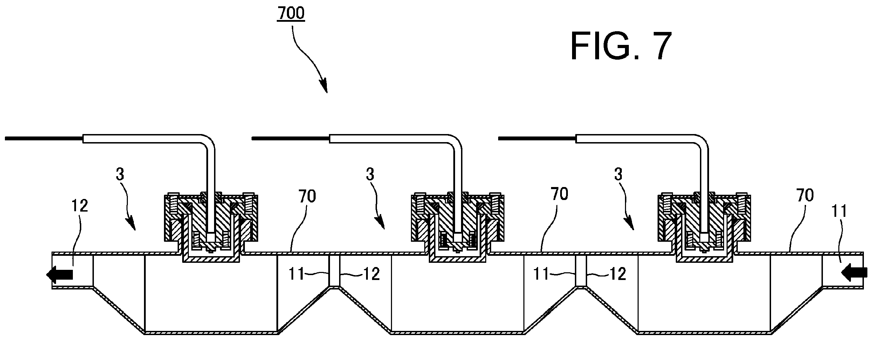

Next, a fluid sterilization unit according to the present invention will be described with reference to FIGS. 5 to 7. The fluid sterilization unit includes a plurality of the fluid sterilization devices connected in series. FIG. 5 is a vertical cross-sectional view of a fluid sterilization unit 500 constituted by a plurality of the fluid sterilization devices 1 according to the first embodiment. FIG. 6 is a vertical cross-sectional view of a fluid sterilization unit 600 constituted by a plurality of the fluid sterilization devices 2 according to the second embodiment. FIG. 7 is a vertical cross-sectional view of a fluid sterilization unit 700 constituted by a plurality of the fluid sterilization devices 3 according to the third embodiment.

As shown in FIG. 5, the fluid sterilization unit 500 is configured by connecting the inlet port 11 of one of the fluid sterilization devices 1 and the outlet port 12 of the adjacent one of the fluid sterilization devices 1 in the adjacent two fluid sterilization devices 1 adjacent to each other. In this manner, the plurality of fluid sterilization devices 1 connected in series can increase the irradiation amount of ultraviolet light to the fluid flowing through the main tube portions 10. As a result, the sterilization efficiency for the fluid can be increased.

Similarly, in the fluid sterilization unit 600 (and also 700), the inlet port 11 of one of the fluid sterilization devices 2 (3) and the outlet port 12 of the adjacent one of the fluid sterilization devices 2 (3) in the two adjacent fluid sterilization devices 2 (3) are connected (see FIGS. 6 and 7). As in the case of the fluid sterilization unit 500, the irradiation amount of ultraviolet light to the fluid flowing through the main tube portions 60 (70) can be increased, and as a result, the sterilization efficiency for the fluid can be increased.

The fluid sterilization device and the fluid sterilization unit according to the present invention are used, for example, in an ultraviolet ray sterilization apparatus, a water purifier, a water heater, a cooling water circulation apparatus, a water server, a drink server, and the like, although the use thereof is not limited to these.

## Claims

1. A fluid sterilization device (1, 2, 3) **characterized by** comprising:
a main tube portion (10, 60, 70) including an inlet port (11) for a fluid provided on one side of both ends opposite to each other in an axis direction of the main tube portion (10, 60, 70) and an outlet port (12) for the fluid provided on the other side;
a communication tube portion (20) connected to a side wall (10S, 10S1) of the main tube portion (10, 60, 70) so as to communicate with an inside of the main tube portion (10, 60, 70);
a light source portion (30) accommodated in the communication tube portion (20) so as to face the inside of the main tube portion (10, 60, 70), the light source portion (30) being configured to irradiate the fluid flowing through the main tube portion (10, 60, 70) with ultraviolet light; and
a cap portion (40) having an ultraviolet ray transmitting ability, the cap portion (40) being configured to separate the light source portion (30) and the main tube portion (10, 60, 70), wherein
the cap portion (40) has a distal end wall (41) that faces the light source portion (30), and
the distal end wall (41) is disposed along the side wall (10S, 10S1) connected to the communication tube portion (20), or is disposed inside the main tube portion (10, 60, 70) from the side wall (10S, 10S1).

2. The fluid sterilization device (2, 3) according to claim 1, **characterized in that:**
the main tube portion (60, 70) includes
a first tube portion (61, 71) that includes a communication region with the communication tube portion (20), and
a second tube portion (62, 72) connected to an end of the first tube portion (61, 72) on a side closer to the inlet port (11); and
the second tube portion (62, 72) has a diameter that increases from a side closer to the inlet port (11) toward the first tube portion (61, 72).

3. The fluid sterilization device (3) according to claim 2, **characterized in that**
in a vertical cross-sectional view of the second tube portion (72), the second tube portion (72) includes
a side wall region (72c) on one side disposed closer to the light source portion (30), the side wall region (72c) on the one side extending in the axis direction of the main tube portion (70), and
a side wall region (72d) on the other side, which faces the side wall region (72c) on the one side, the side wall region (72d) being inclined so as to increase the distance to the side wall region (72c) on the one side as approaching the first tube portion (71) from the inlet port (11) side.

4. A fluid sterilization unit (500, 600, 700) **characterized by** comprising:
a plurality of the fluid sterilization devices (1, 2, 3) according to any one of claims 1 to 3, wherein
two adjacent fluid sterilization devices (1, 2, 3) of these are configured such that an inlet port (11) of one of the two adjacent fluid sterilization devices (1, 2, 3) and an outlet port (12) of the other of the two adjacent fluid sterilization devices (1, 2, 3) are connected to each other.
